(19) Europäisches Patentamt
European Patent Office
Office européen des brevets

(11) Veröffentlichungsnummer: **0 226 973 B1**

# (12) EUROPÄISCHE PATENTSCHRIFT

(45) Veröffentlichungstag der Patentschrift: **06.03.91**

(51) Int. Cl.[5]: **C07C 215/08**, A61K 7/13

(21) Anmeldenummer: **86117311.0**

(22) Anmeldetag: **12.12.86**

(54) **4-Bis(2'-hydroxyethyl)amino-1-(3'-hydroxypropyl)amino-2-nitro-benzol, Verfahren zu dessen Herstellung sowie Mittel zum Färben von Haaren.**

(30) Priorität: **23.12.85 DE 3545994**

(43) Veröffentlichungstag der Anmeldung:
**01.07.87 Patentblatt 87/27**

(45) Bekanntmachung des Hinweises auf die Patenterteilung:
**06.03.91 Patentblatt 91/10**

(84) Benannte Vertragsstaaten:
**AT CH ES GR IT LI SE**

(56) Entgegenhaltungen:
**FR-A- 1 581 135**
**GB-A- 2 112 818**
**GB-A- 2 164 656**
**GB-A- 2 164 959**

(73) Patentinhaber: **Wella Aktiengesellschaft**
**Berliner Allee 65**
**W-6100 Darmstadt(DE)**

(72) Erfinder: **Braun, Hans-Jürgen, Dr.**
**Im Dorf 32**
**CH-3183 Albligen(CH)**
Erfinder: **Konrad, Eugen**
**Mecklenburger Strasse 101**
**W-6100 Darmstadt(DE)**
Erfinder: **Mager, Herbert, Dr.**
**Beaumont 5**
**CH-1700 Fribourg(CH)**

**EP 0 226 973 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft die neue Verbindung der Formel (I) und mit Hilfe von (I) hergestellte Mittel zum Färben von Haaren.

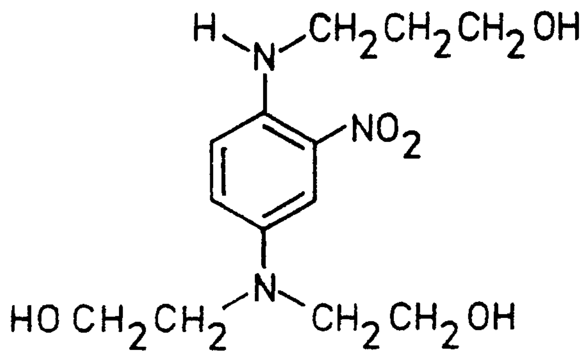

(I)

Menschliche Haare können mit Oxidationshaarfärbemitteln in fast beliebigen Farbnuancen gefärbt werden. Für diesen Färbevorgang werden zur Entwicklung der Farbstoffe Oxidationsmittel, wie zum Beispiel Hydrogenperoxid, benötigt.

Durch die Einwirkung des Hydrogenperoxids auf das Haar wird dieses gebleicht, was für die Erzielung bestimmter Farbnuancen unerwünscht ist. Gleichzeitig wird das Haar in seiner Struktur geschädigt. Es besteht daher ein Bedarf nach Mitteln zum Färben von menschlichen Haaren, bei denen die Anwendung von Oxidationsmitteln nicht notwendig ist.

Diese Aufgabe kann durch die Anwendung von direkt auf das Haar aufziehenden Farbstoffen gelöst werden. Als direktziehende Farbstoffe finden heute Nitrofarbstoffe in Haarfärbemitteln eine breite Anwendung. Durch die Kombination mehrerer verschieden gefärbter Nitrofarbstoffe lassen sich Haare ohne die Verwendung von Oxidationsmitteln in natürlichen bis modischen Farbnuancen einfärben.

Dabei lassen sich natürliche Haarfarben durch die Kombination eines blauen mit einem orangen Farbstoff oder durch die Kombination eines blauen mit einem rein gelben und einem roten Nitrofarbstoff herstellen.

Es besteht daher in jedem Falle ein Bedarf nach einem blauen Nitrofarbstoff, an den aber noch viele weitere Anforderungen gestellt werden. So muß er in toxikologischer und dermatologischer Hinsicht unbedenklich sein und die Erzielung von Färbungen in der gewünschten Intensität ermöglichen, was unter anderem auch eine ausreichende Wasserlöslichkeit voraussetzt. Andererseits dürfen die Farbstoffe bei der Nachbehandlung oder bei der Haarwäsche nicht zu leicht wieder ausgewaschen werden. Außerdem wird für die erzielten Haarfärbungen eine gute Licht-, Säure- und Reibechtheit gefordert.

Zur Erzielung blauer Nitrofarbstoffe werden in der Regel trialkylierte 2-Nitro-p-phenylen-diaminderivate der allgemeinen Formel (II) verwendet

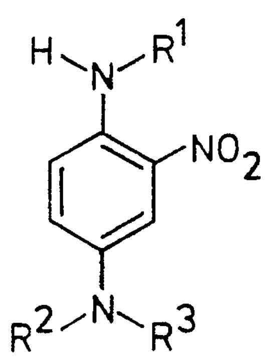

(II)

Es hat bereits zahlreiche Versuche gegeben, blaue Nitrofarbstoffe herzustellen, die den oben beschriebenen Anforderungen entsprechen. So wird das in der DE-OS I 569 808 beschriebene 4-Bis(2'-hydroxyethyl) amino-I-ethylamino-2-nitro-benzol (III)

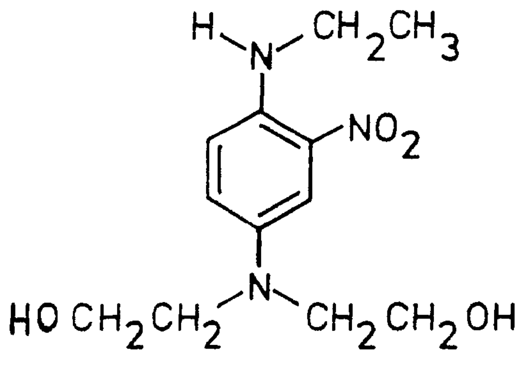

(III)

in Haarfärbemitteln eingesetzt. Die ohne Zusatz von Lösungsvermittlern schlechte Löslichkeit des Farbstoffes von unter 0,l5 Gewichtsprozent in Wasser verhindert jedoch einen Einsatz der Verbindung in Emulsionssystemen in den Mengen, die zur Erzielung einer hohen Farbtiefe erforderlich sind.

Im Gegensatz hierzu führt die höhere Wasserlöslichkeit des in der DE-PS I 0l7 750 genannten 4-Bis(2′-hydroxyethyl)amino-l-(2′-hydroxyethyl)amino-2-nitro-benzols (IV)

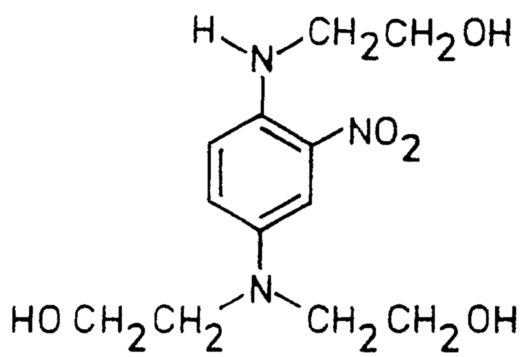

(IV)

zur guten Anwendbarkeit bei hohen Konzentrationen. Der Farbstoff wird jedoch beim Waschen der Haare sehr schnell wieder ausgespült, und die Farbechtheit ist ebenfalls nicht zufriedenstellend.

Die vorstehend beschriebenen Probleme in anwendungstechnischer Hinsicht treten bei der Verwendung des 4-Bis-(2′-hydroxyethyl)amino-l-methylamino-2-nitro-benzols (V)

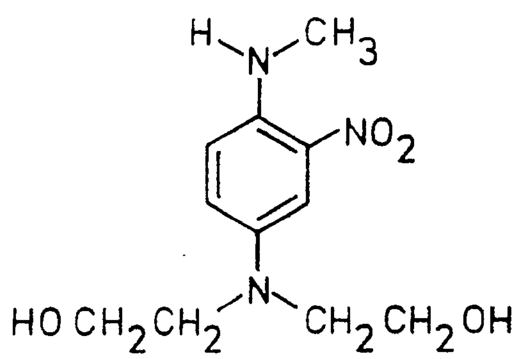

(V)

nach der DE-PS I 299 002 weniger auf. Lediglich die Erzielung dunkler Nuancen kann durch die geringe Wasserlöslichkeit von nur etwa 0,5 Gewichtsprozent erschwert werden. Die toxikologischen Eigenschaften des Farbstoffes (V) sind jedoch sehr unbefriedigend.

Umso überraschender ist es nach dem zuvor Gesagten, daß der neue blaue Nitrofarbstoff 4-Bis(2′-hydroxyethyl)amino-l-(3′-hydroxypropyl)amino-2-nitro-benzol (I)

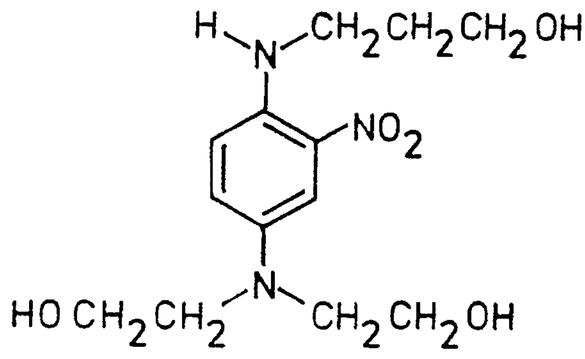

(I),

der ein Homologes zu (IV) darstellt, teilweise andere Eigenschaften aufweist, die sich aus den bekannten Verbindungen (III) bis (V) nicht ableiten lassen und die den oben erwähnten Anforderungen in idealer Weise genügen.

So ist die Färbung der Haare mit dem neuen Farbstoff (I) viel intensiver als die Färbung, die mit dem Farbstoff (IV) erhalten wird. Dies ist ein überraschender Befund, insbesondere wenn man in Betracht zieht, daß der molare Extinktionskoeffizient der Absorptionsbande im sichtbaren Bereich des Ultraviolett-Spektrums für die beiden Verbindungen (I) und (IV) gleich groß ist.

Darüberhinaus enthalten die Färbungen, die mit dem neuen Farbstoff (I) erzielt werden, weniger Rotanteil als die Färbungen mit dem Farbstoff (IV). Dies ist auch aus den Ultraviolett-Spektren der beiden Verbindungen ersichtlich. Während das Maximum der Absorptionsbande im sichtbaren Bereich bei der Verbindung (IV) bei 532 nm liegt, ist das Maximum bei Verbindung (I) deutlich zum Blauen hin auf 543 nm verschoben. Dieses Verhalten ist überraschend und aus dem Stand der Technik nicht ableitbar. Der neue Farbstoff (I) bringt somit einen erheblichen Vorteil bei der Erzeugung von natürlichen Farbnuancen, bei denen ein blauer Farbstoff mit zu hohem Rotanteil nicht verwendbar ist.

Die Löslichkeit des neuen Farbstoffes (I) in Wasser beträgt etwa 2 Gewichtsprozent. Demgegenüber beträgt die Löslichkeit des bekannten Farbstoffes (IV) nur etwa I Gewichtsprozent. Die bessere Wasserlöslichkeit von (I) wirkt sich aber in erstaunlicher Weise nicht auf die erzielbare Farbintensität in den Haarfärbungen aus. Man erhält bei Färbungen mit äquimolaren Konzentrationen mit dem neuen Farbstoff (I) deutlich intensivere Farbtöne als mit Farbstoff (IV). Wegen der besseren Wasserlöslichkeit von Farbstoff (I) neigt dieser aber auch bei höheren Konzentrationen nicht zur Kristallisation. Somit ist es prinzipiell möglich, mit (I) auch dunklere Töne zu erzeugen als mit dem bekannten Farbstoff (IV). Der neue Farbstoff (I) besitzt also für Haarfärbezwecke viel günstigere Eigenschaften als die bekannte Verbindung (IV).

Endlich entscheiden für die Verwendung eines Farbstoffes auch dessen toxikologische Eigenschaften. Hierzu wurde nun gefunden, daß der neue Farbstoff (I) sowohl in Mutagenitätstest nach Ames als auch im Human-Lymphozytentest im Gegensatz zu Farbstoff (V) keine mutagene Wirkung zeigt, und somit toxikologisch erheblich besser als Farbstoff (V) abschneidet.

Ein Syntheseweg für die Herstellung von Verbindungen, die eine ähnliche Struktur wie das 4-Bis(2'-hydroxyethyl)-amino-I-(3'-hydroxypropyl)amino-2-nitro-benzol (I) besitzen, ist in der DE-PS I 768 999 beschrieben. Entgegen dem in der DE-PS I 768 999 offenbarten Verfahren wurde nun jedoch gefunden, daß es in bezug auf die Ausbeute viel günstiger ist, wenn man im ersten Reaktionsschritt ausgehend vom 4-Fluor-3-nitro-anilin dieses mit 3-Amino-propanol bei etwa I00 °C zum 4-(3'-Hydroxypropyl)amino-3-nitro-anilin umsetzt. Im zweiten Reaktionsschritt wird dann das 4-(3'-Hydroxypropyl)amino-3-nitroanilin mit 2-Chlor-ethanol in Gegenwart einer Base bei II0 -I20 °C zum Endprodukt (I) umgesetzt. Als Base kann hierbei eine wäßrige Lösung eines Alkalihydroxids, ein Oxid oder ein Hydroxid eines Erdalkalimetalls oder aber auch ein organisches Amin, wie zum Beispiel Triethylamin, verwendet werden. Der neue Farbstoff wird zweckmäßigerweise durch Umkristallisation aus Essigsäureethylester gereinigt.

Gegenstand der vorliegenden Anmeldung sind ferner Mittel zur Färbung von Haaren mit einem Farbstoffgehalt und üblichen kosmetischen Zusätzen, dadurch gekennzeichnet, daß sie die Verbindung der Formel (I) oder deren physiologisch verträgliches Salz, wie zum Beispiel das Hydrochlorid, das Sulfat oder das Acetat, enthalten. Die erfindungsgemäßen Mittel zur Färbung von Haaren betreffen insbesondere solche, die ohne Zugabe eines Oxidationsmittels angewendet werden. Solche Haarfärbemittel enthalten neben dem erfindungsgemäßen blauen Nitrofarbstoff der angegebenen Formel (I) noch andere direkt auf das Haar aufziehende Farbstoffe. Von diesen für die Haarfärbung bekannten Farbstoffen seien beispielsweise die folgenden Klassen erwähnt:

Aromatische Nitrofarbstoffe, wie zum Beispiel 2-Amino-4-nitro-phenol, 2-(2'-Hydroxyethyl)amino-4,6-dinitro-phenol, 3-Nitro-4-(2′,3′-dihydroxypropyl)amino-trifluor-methylbenzol, l-(2′-Hydroxyethyl)amino-2-amino-4-nitrobenzol, 2-Nitro-p-phenylendiamin, 5-Chlor-4-(2′-hydroxyethyl)amino-2-nitro-anilin, l-Amino-4-(2′,3′-dihydroxypropyl)amino-5-chlor-2-nitro-benzol, 4-(2′-Ureidoethyl)amino-nitrobenzol, l-Amino-2-(2′-hydroxyethyl)amino-5-nitro-benzol, l-Amino-2-nitro-4-bis(2′-hydroxyethyl)amino-benzol, Pikraminsäure, 4-(2′,3′-Dihydroxypropyl)amino-3-nitro-toluol, 4-(2′-Hydroxyethyl)amino-3-nitro-chlorbenzol; Azofarbstoffe, wie zum Beispiel Acid Brown 4 (C. I. l4 805); Anthrachinonfarbstoffe, wie zum Beispiel Disperse Blue 23 (C. I. 6l 545), Disperse Violet 4 (C. I. 6l l05), l,4,5,8-Tetraamino-anthrachinon und l,4-Diamino-anthrachinon; Triphenylmethanfarbstoffe, wie zum Beispiel Basic Violet l (C. I. 42 535), wobei die Farbstoffe dieser Klassen je nach der Art ihrer Substituenten sauren, nichtionogenen oder basischen Charakter haben können. Weitere geeignete direkt auf das Haar aufziehende Farbstoffe sind beispielsweise in dem Buch von J. C. Johnson, "Hair Dyes" Noyes Data Corp. Park-Ridge (USA) (l973), beschrieben.

Mit Haarfärbemitteln, die derartige Gemische von Farbstoffen enthalten, lassen sich Naturtöne, modische Blond- und Brauntöne und leuchtende Modetöne herstellen.

Die Zubereitungsform der hier beschriebenen Haarfärbemittel auf der Basis von direkt auf das Haar aufziehenden Farbstoffen kann beispielsweise eine Lösung, insbesondere eine wäßrige oder wäßrig-alkoholische Lösung, sein. Bevorzugte Zubereitungsformen sind weiterhin eine Creme, ein Gel oder eine Emulsion, wobei sie auch im Gemisch mit einem Treibgas oder mittels einer Pumpe versprüht werden können.

Übliche Zusätze in Lösungen, Cremes, Emulsionen oder Gelen sind zum Beispiel Lösungsmittel wie Wasser, niedere aliphatische Alkohole, beispielsweise Ethanol, Propanol, Isopropanol, Glycerin oder Glykole wie Ethylenglykol und Propylenglykol, oder auch Glykolether, weiterhin Netzmittel oder Emulgatoren aus den Klassen der anionischen, kationischen, amphoteren oder nichtionogenen oberflächenaktiven Substanzen wie Fettalkoholsulfate, Fettalkoholethersulfate, Alkylsulfonate, Alkylbenzolsulfate, Alkyltrimethylammoniumsalze, Alkylbetaine, oxethylierte Fettalkohole, oxethylierte Nonylphenole, Fettsäurealkanolamide, oxethylierte Fettsäureester, ferner Verdicker wie höhere Fettalkohole, Bentonit, Stärke, Polyacrylsäure, Cellulosederivate, wie zum Beispiel Carboxymethylcellulose, ferner Alginate, Vaseline, Paraffinöl und Fettsäuren sowie außerdem Pflegestoffe wie Lanolinderivate, Cholesterin, Panthothensäure und Betain, weiterhin Parfümöle und Komplexbildner. Die erwähnten Bestandteile werden in den für solche Zwecke üblichen Mengen verwendet, zum Beispiel die Netzmittel und Emulgatoren in Konzentrationen von etwa 0,5 bis 30 Gewichtsprozent, während die Verdicker in einer Menge von etwa 0,l bis 25 Gewichtsprozent in den Zubereitungen enthalten sein können.

Der Farbstoff der Formel (I) soll in diesen Haarfärbemitteln in einer Konzentration von etwa 0,0l bis 2,0 Gewichtsprozent, vorzugsweise von 0,0l bis l,0 Gewichtsprozent, enthalten sein. Der Gesamtgehalt an Farbstoffen liegt in den Grenzen von etwa 0,0l bis 4,0 Gewichtsprozent.

Der pH-Wert dieser Färbemittel liegt im Bereich von 3 bis l2, insbesondere bei pH 8 bis ll,5, wobei die Einstellung des gewünschten pH-Wertes hauptsächlich mit Ammoniak erfolgt, jedoch auch mit organischen Aminen wie beispielsweise Mono-, Di- oder Triethanolamin vorgenommen werden kann.

Ihre Anwendung erfolgt in üblicher Weise durch Aufbringen einer für die Haarfärbung ausreichenden Menge des Mittels auf die Haare, mit denen es eine Zeitlang, vorzugsweise zwischen 5 und 30 Minuten, in Berührung bleibt. Anschließend werden die Haare mit Wasser, gegebenenfalls noch mit einer schwachen organischen Säure, gespült und sodann getrocknet. Als schwache organische Säure können beispielsweise Essigsäure, Zitronensäure, Weinsäure und ähnliche Verwendung finden.

Die vorstehend beschriebenen Haarfärbemittel können selbstverständlich auch kosmetische Polymerisate enthalten, wodurch gleichzeitig mit der Färbung eine Festigung der Haare erreicht wird. Solche Mittel werden im allgemeinen als Tönungsfestiger oder Farbfestiger bezeichnet.

Von den für diesen Zweck in der Kosmetik bekannten Polymerisaten seien beispielsweise Polyvinylpyrrolidon, Polyvinylacetet, Polyvinylalkohol oder Polyacrylverbindungen wie Acrylsäure- beziehungsweise Methacrylsäurepolymerisate, basische Polymerisate von Estern aus diesen beiden Säuren und Aminoalkoholen beziehungsweise deren Salze oder Quaternisierungsprodukte, Polyacrylnitril, Polyvinyllactame sowie Copolymerisate aus derartigen Verbindungen wie z.B. Polyvinylpyrrolidon-Vinylacetat erwähnt.

Auch natürliche Polymere, wie Chitosan (entacetyliertes Chitin) oder Chitosanderivate, können für den genannten Zweck eingesetzt werden.

Die Polymerisate sind in diesen Mitteln in der üblichen Menge von etwa l bi 5 Gewichtprozent enthalten. Die pH-Werte der Mittel liegen im Bereich von etwa 6,0 bis 9,5.

Die Anwendung dieser Haarfärbemittel mit zusätzlicher Festigung erfolgt in bekannter und üblicher Weise durch Befeuchten des Haares mit dem Festiger, Festlegen (Einlegen) des Haares zur Frisur und anschließende Trocknung.

Selbstverständlich können die vorstehend beschriebenen Haarfärbemittel gegebenenfalls weitere gebräuchliche kosmetische Zusätze wie zum Beispiel Pflegestoffe, Netzmittel, Verdicker, Weichmacher, Konservierungsstoffe, Antischuppenwirkstoffe und Parfümöle enthalten.

**Haarfärbebeispiele**

Beispiel 1 Haarfärbemittel in flüssiger Form

| | |
|---|---|
| 0,6 g | 4-Bis(2'-hydroxyethyl)amino-1-(3'-hydroxypropyl)amino-2-nitro-benzol-hydrochlorid |
| 0,5 g | Hydroxyethylcellulose |
| 5,0 g | Laurylalkohol-diglykolethersulfat-Natriumsalz, 28 prozentige wäßrige Lösung |
| 15,0 g | Isopropanol |
| 0,5 g | Ammoniak, 25 prozentige wäßrige Lösung |
| 78,4 g | Wasser, vollentsalzt |
| 100,0 g | |

Das Haarfärbemittel wird auf weiße menschliche Haare aufgetragen und l0 Minuten lang einwirken gelassen. Nach Spülung mit Wasser und Trocknung ist das Haar dunkelblau gefärbt.

Beispiel 2 Farbfestiger

| | |
|---|---|
| 0,02 g | 4-Bis(2'-hydroxyethyl)amino-1-(3'-hydroxypropyl)amino-2-nitro-benzol |
| 2,00 g | Polyvinylpyrrolidon |
| 0,10 g | Glycerin |
| 40,00 g | Isopropanol |
| 57,88 g | Wasser, vollentsalzt |
| 100,00 g | |

Weiße menschliche Haare werden mit der festigenden Färbelösung zur Frisur eingelegt und getrocknet. Das Haar zeigt danach einen silber-blauen Schimmer.

Beispiel 3 Haarfärbemittel in Gelform

| | |
|---|---|
| 1,0 g | 4-Bis(2'-hydroxyethyl)amino-1-(3'-hydroxy-propyl)amino-2-nitro-benzol |
| 0,1 g | 1-Amino-2-nitro-4-(2'-hydroxyethyl)amino-5-chlor-benzol |
| 1,0 g | 3-Nitro-4-(2',3'-dihydroxypropyl)amino-trifluormethylbenzol |
| 17,5 g | Ölsäure |
| 7,5 g | Isopropanol |
| 7,0 g | Diethanolamin |
| 65,9 g | Wasser, vollentsalzt |
| 100,0 g | |

Das Haarfärbemittel wird auf graue menschliche Haare aufgetragen und und 20 Minuten lang einwirken gelassen. Nach dem Spülen mit Wasser und dem Trocknen ist das Haar braun mit purpurroten Reflexen gefärbt.

Beispiel 4 Haarfärbemittel in Cremeform

| | |
|---|---|
| 1,8 g | 4-Bis(2'-hydroxyethyl)amino-1-(3'-hydroxy-propyl)amino-2-nitro-benzol-hydrochlorid |
| 0,4 g | 1-(2'-Hydroxyethyl)amino-2-amino-4-nitro-benzol |
| 0,2 g | 2-Nitro-p-phenylendiamin-hydrochlorid |
| 7,5 g | Cetylalkohol |
| 1,8 g | Laurylalkohol-diglykolethersulfat-Natrium-salz, 28 prozentige wäßrige Lösung |
| 0,1 g | p-Hydroxybenzoesäuremethylester |
| 0,6 g | Ammoniak, 25 prozentige wäßrige Lösung |
| 87,6 g | Wasser, vollentsalzt |
| 100,0 | |

50 g des vorstehenden Haarfärbemittels werden auf graue menschliche Haare aufgetragen und nach einer Einwirkungszeit von 20 Minuten mit Wasser ausgespült. Nach der Trocknung ist das Haar in einem natürlichen braunen Farbton gefärbt.

**Herstellungsbeispiel**

I. Stufe 4-(3'-Hydroxypropyl)amino-3-nitro-anilin

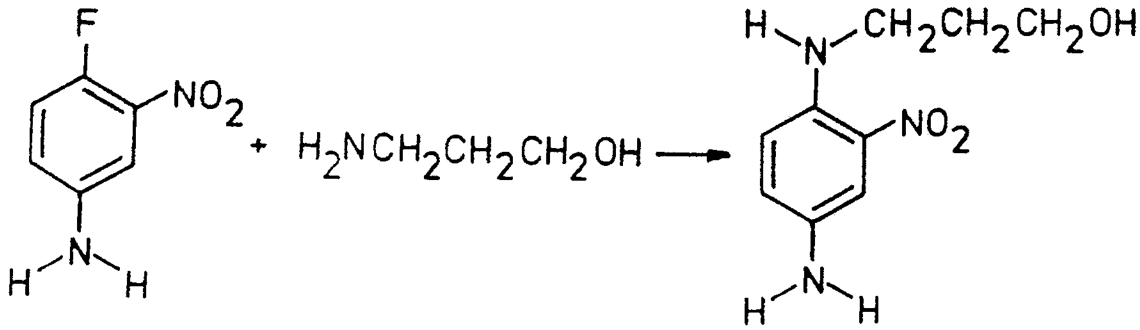

156 g (1,0 mol) 4-Fluor-3-nitro-anilin werden in 1500 g (20,0 mol) 3-Amino-propanol gelöst und die Lösung 30 bis 60 Minuten lang auf l00° C erwärmt, bis die dünnschichtchromatographische Kontrolle kein Ausgangsprodukt mehr anzeigt. Das Reaktionsprodukt wird dann mit 4,5 l Wasser ausgefällt, abgesaugt, mit Wasser gewaschen und getrocknet. Man erhält 200 g (95 % der Theorie) der Hydroxypropylaminoverbindung in Form dunkelbrauner Nadeln mit grünlichem Glanz mit dem Schmelzpunkt von l08 - l09° C.

| CHN-Analyse: | | % C | % H | % N |
|---|---|---|---|---|
| $C_9H_{13}N_3O_3$ | berechnet: | 51,18 | 6,20 | 19,89 |
| | gefunden: | 51,21 | 6,24 | 19,80 |

2. Stufe

4 Bis(2'- hydroxyethyl)amino-l-(3'-hydroxypropyl)amino-2-nitro-benzol (I)

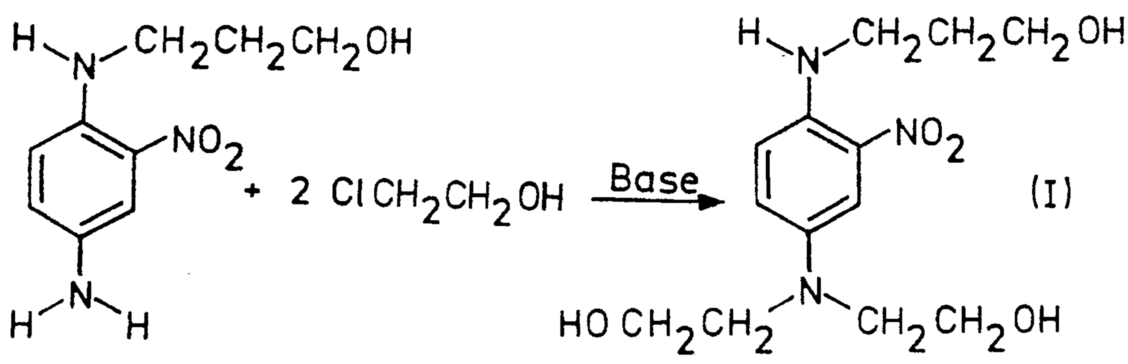


(I)

42 g (0,2 mol) 4-(3'-Hydroxypropyl)amino-3-nitro-anilin werden in 750 ml 2-Chlor-ethanol gelöst und die Lösung auf ll0 bis l20° C erwärmt. Dann werden 200 ml 2-normale Natronlauge mit einer solchen Geschwindigkeit zugetropft, daß gewährleistet ist, daß das Reaktionsgemisch während der Umsetzung stets einen alkalischen pH-Wert aufweist. Der Fortgang der Reaktion wird mit Dünnschichtchromatographie kontrolliert. Nach vollständiger Umsetzung des Ausgangsprodukts wird das überschüssige 2-Chlor-ethanol abdestilliert. Anschließend wird der Rückstand mit gesättigter wäßriger Kochsalzlösung versetzt und mit Essigsäureethylester extrahiert. Die Extraktionslösung wird über Magnesiumsulfat getrocknet und ein Teil des Lösungsmittels abdestilliert. Nach Animpfen kristallisiert das Produkt aus. Es wird abgesaugt, mit Diethylether gewaschen und getrocknet. Man erhält 4l g (70 % der Theorie) des 4-Bis(2'-hydroxyethyl)-amino-l-(3'-hydroxypropyl)amino-2-nitro-benzols (I) in Form dunkelblauer Kristalle mit grünlichem Glanz mit dem Schmelzpunkt von 76 bis 77° C.

| CHN-Analyse | | % C | % H | % N |
|---|---|---|---|---|
| $C_{13}H_{21}N_3O_5$ | berechnet: | 52,16 | 7,07 | 14,04 |
| | gefunden: | 52,19 | 7,06 | 14,02 |

In der 2. Stufe des hier beschriebenen Verfahrens zur Herstellung des Farbstoffes (I) können auch andere Basen eingesetzt werden.

a) Die Herstellung von (I) wird wie in der Stufe 2 beschrieben durchgeführt, mit der Ausnahme, daß anstatt der Natronlauge 75 g Calciumoxid als Base zugesetzt werden. Nach vollständiger Reaktion wird das Calciumoxid durch Filtration entfernt. Die weitere Aufarbeitung erfolgt wie oben beschrieben.

b) Die Herstellung von (I) wird wie in der Stufe 2 beschrieben durchgeführt, mit der Ausnahme, daß anstatt der Natronlauge 60 g Triethylamin zugetropft werden. Die Aufarbeitung erfolgt wie oben beschrieben.

3. Stufe

4-Bis(2′-hydroxyethyl)amino-l-(3′-hydroxypropyl)amino-2-nitro-benzol-hydrochlorid

Die freie Base (I) wird in Ethanol aufgelöst. Nach der Zugabe von 3-molarar Salzsäure in Ethanol fällt das Hydrochlorid von (I) als gelbes Pulver aus. Es wird abgesaugt und getrocknet. Das Hydrochlorid ist hygroskopisch.

| CHNCl-Analyse | | % C | % H | % Cl | % N |
|---|---|---|---|---|---|
| $C_{13}H_{22}ClN_3O_5$ | berechnet: | 46,50 | 6,60 | 10,56 | 12,51 |
| | gefunden: | 46,37 | 6,72 | 10,49 | 12,25 |

Alle Prozentangaben dieser Anmeldung stellen, soweit nicht anders angegeben, Gewichtsprozente dar.

**Ansprüche**

**1.** 4-Bis(2′′-hydroxyethyl)amino-l-(3′′ hydroxypropyl)amino-2-nitro-benzol der Formel (I)

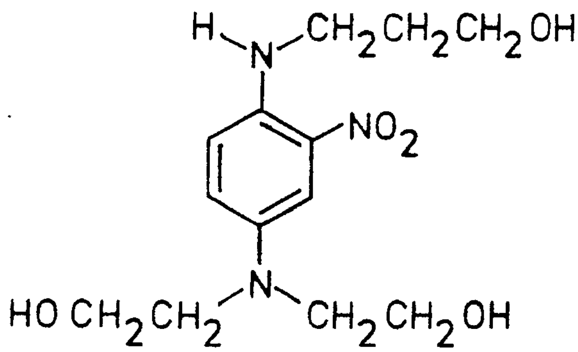

(I)

**2.** Mittel zur Färbung von Haaren mit einem Farbstoffgehalt und üblichen kosmetischen Zusätzen, dadurch gekennzeichnet, daß es die Verbindung nach Anspruch I oder deren physiologisch verträgliches Salz enthält.

3. Mittel nach Anspruch 2, dadurch gekennzeichnet, daß es 0,0I bis 2,0 Gewichtsprozent des Farbstoffes nach Anspruch I enthält.

4. Mittel nach den Ansprüchen 2 und 3, dadurch gekennzeichnet, daß es zusätzlich bekannte, direkt auf das Haar aufziehende Farbstoffe, die ausgewählt sind aus 2-Amino-4-nitro-phenol, 2-(2'-Hydroxyethyl)-amino-4,6-dinitro-phenol, 3-Nitro-4-(2',3'-dihydroxypropyl)amino-trifluormethylbenzol, I-(2'-Hydroxyethyl)amino-2-amino-4-nitro-benzol, 2-Nitro-p-phenylendiamin, 5-Chlor-4-(2'-hydroxyethyl)amino-2-nitro-anilin, I-Amino-4-(2',3'-dihydroxypropyl)amino-5-chlor-2-nitrobenzol, 4-(2'-Ureidoethyl)amino-nitrobenzol, I-Amino-2-(2' hydroxyethyl)amino-5-nitro-benzol, I-Amino-2-nitro-4-bis(2'-hydroxyethyl)amino-benzol, Pikraminsäure, 4-(2',3 -Dihydroxypropyl)amino-3-nitro-toluol, 4-(2'-Hydroxyethyl)amino-3-nitro-chlorbenzol, Basic Violet I (C. I. 42 535), Acid Brown 4 (C. I. I4 805), Disperse Violet 4 (C. I. 6I I05), Disperse Blue 23 (C. I. 6I 545), I,4,5,8-Tetraamino-anthrachinon und I,4-Diamino-anthrachinon, enthält.

5. Mittel nach den Ansprüchen 2 bis 4, dadurch gekennzeichnet, daß der Gesamtgehalt an Farbstoffen 0,0I bis 4,0 Gewichtsprozent beträgt.

6. Mittel nach den Ansprüchen 2 bis 5, dadurch gekennzeichnet, daß es einen pH-Wert von 3 - I2, vorzugsweise 8 - II,5, aufweist.

7. Mittel nach den Ansprüchen 2 bis 6, dadurch gekennzeichnet, daß der alkalische pH-Wert mit Ammoniak, Monoethanolamin, Diethanolamin oder Triethanolamin eingestellt ist.

8. Mittel nach den Ansprüchen 2 bis 7, dadurch gekennzeichnet, daß es zwecks Haarfestigung in wäßrig-alkoholischer Lösung mindestens ein kosmetisches Polymerisat enthält.

9. Mittel nach den Ansprüchen 2 bis 8, dadurch gekennzeichnet, daß es weitere kosmetische Zusätze, wie Pflegestoffe, Netzmittel, Verdicker, Weichmacher und Parfümöle, enthält.

10. Verfahren zur Herstellung der Verbindung nach Anspruch I, dadurch gekennzeichnet, daß man 4-Fluor-3-nitro-anilin mit 3-Amino-propanol umsetzt und anschließend das entstandene 4-(3' Hydroxypropyl)-amino-3-nitro-anilin mit 2-Chlor-ethanol in Gegenwart einer Base oxethyliert.

**Claims**

1. 4-bis(2'-hydroxyethyl) amino-1-(3'-hydroxypropyl)-amino-2-nitro-benzene of formula (I):

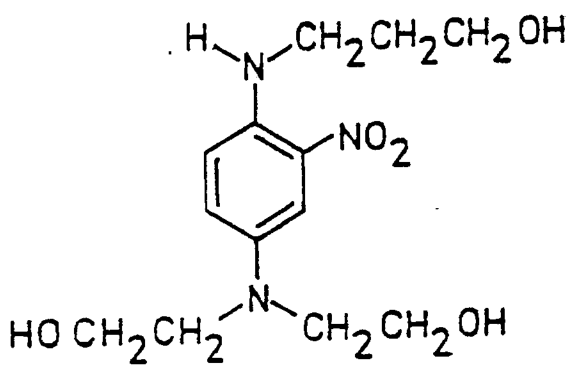

(I)

2. Hair colouring agent containing dye and conventional cosmetic additives, characterized in that it contains the compound according to claim 1 or the physiologically compatible salt thereof.

3. Agent according to claim 2, characterised in that it contains 0.01 to 2.0 weight percent of the dye according to claim 1.

4. Agent according to claims 2 and 3, characterised in that it additionally contains known dyes which can

be applied directly to the hair and are selected from 2-amino-4-nitro-phenol, 2-(2'-hydroxyethyl)amino-4,6-dinitro-Phenol, 3-nitro-4-(2',3'-dihydroxypropyl)amino-trifluoromethylbenzene, 1-(2'-hydroxyethyl)-amino-2-amino-4-nitro-benzene, 2-nitro-p-phenylenediamine, 5-chloro-4-(2'-hydroxyethyl)amino-2-nitro-aniline. 1-amino-4-(2',3'-dihydroxypropyl)amino-5-chloro-2-nitro-benzene, 4-(2'-ureidoethyl)amino-nitrobenzene, 1-amino-2-(2'-hydroxyethyl)amino-5-nitro-benzene, 1-amino-2-nitro-4-bis(2'-hydroxyethyl)-amino-benzene, picramic acid, 4-(2',3'-dihydroxypropyl)amino-3-nitro-toluene, 4-(2'-hydroxyethyl)amino-3-nitro-chlorobenzene, Basic Violet 1 (C.I. 42 535), Acid Brown 4 (C.I. 14 805), Disperse Violet 4 (C.I. 61 105), Disperse Blue 23 (C.I. 61 545), 1,4,5,8-tetraamino-anthraquinone and 1,4-diamino-anthraquinone.

5. Agent according to claims 2 to 4, characterised in that the total content of dye is 0.01 to 4.0 weight percent.

6. Agent according to claims 2 to 5, characterised in that it has a pH value of 3 - 12 and preferably 8 - 11.5.

7. Agent according to claims 2 to 6, characterised in that the alkaline pH value is adjusted using ammonia, monoethanolamine, diethanolamine or triethanolamine.

8. Agent according to claims 2 to 7, characterised in that it contains at least one cosmetic polymer in an aqueous-alcohol solution in order to set the hair.

9. Agent according to claims 2 to 8, characterised in that it contains further cosmetic additives such as treatment substances, wetting agents, thickeners, softeners and perfume oils.

10. Process for producing the compound according to claim 1, characterised in that 4-fluoro-3-nitro-aniline is reacted with 3-amino-propanol and subsequently the resultant 4-(3'-hydroxypropyl)-amino-3-nitro-aniline is oxyethylated with 2-chloro-ethanol in the presence of a base.

## Revendications

1. 4-bis(2'-hydroxyéthyl)amino-1-(3'-hydroxypropyl)-amino-2-nitrobenzène répondant à la formule (I)

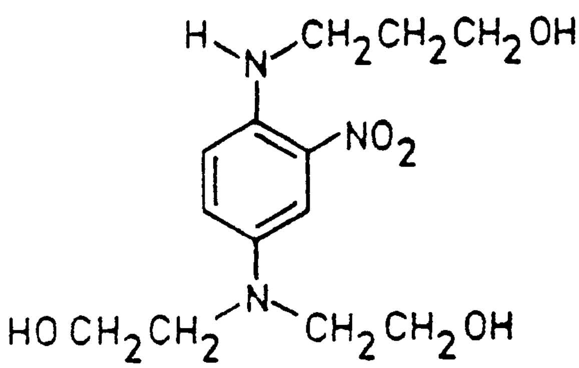

(I)

2. Produit de coloration des cheveux renfermant des colorants et des additifs cosmétiques usuels, caractérisé en ce qu'il renferme le composé selon la revendication 1 ou son sel physiologiquement compatible.

3. Produit selon la revendication 2, caractérisé en ce qu'il renferme 0,01 à 2,0 % en poids du colorant selon la revendication 1.

4. Produit selon les revendications 2 et 3, caractérisé en ce qu'il renferme en outre des colorants à montée directe sur les cheveux connus qui sont choisis parmi le 2-amino-4-nitrophénol, le 2-(2'-hydroxyéthyl)-amino-4,6-dinitrophénol, le 3-nitro-4-(2',3'-dihydroxypropyl)amino-trifluorométhylbenzène, le 1-(2'-hydroxyéthyl)-amino-2-amino-4-nitrobenzène, la 2-nitro-p-phénylène-diamine, la 5-chloro-4-(2'-hydroxyéthyl)amino-2-nitro-aniline, le 1-amino-4-(2',3'-dihydroxypropyl)amino-5-chloro-2-nitrobenzène, le 4-(2'-uréidoéthyl)amino-nitrobenzène, le 1-amino-2-(2'-hydroxyéthyl)amino-5-nitrobenzène, le 1-amino-2-nitro-4-bis(2'-hydroxyéthyl)aminobenzène, l'acide picramique, le 4-(2',3'-dihydroxypropyl)-amino-3-nitrotoluène, le 4-(2'-hydroxyéhtyl)amino-3-nitrochlorobenzène, Basic Violet 1 (I.C. 42 535), Acid Brown 4 (I.C. 14 805), Disperse Violet 4 (I.C. 61 105), Disperse Blue 23 (I.C. 61 545), la 1,4,5,8-tétramino-anthraquinone et la 1,4-diamino-anthraquinone.

**5.** produit selon les revendications 2 à 4, caractérisé en ce que la teneur totale en colorants est de 0,01 à 4,0 % en poids.

**6.** Produit selon les revendications 2 à 5, caractérisé en ce qu'il présente une valeur de pH de 3 à 12, de préférence de 8 à 11,5.

**7.** Produit selon les revendications 2 à 6, caractérisé en ce que la valeur de pH alcaline est ajustée avec de l'ammoniaque, de la monoéthanolamine, de la diéthanolamine ou de la triéthanolamine.

**8.** Produit selon les revendications 2 à 7, caractérisé en ce que, pour la fixation des cheveux, il renferme, en solution dans l'eau et l'alcool, au moins un produit de polymérisation cosmétique.

**9.** Produit selon les revendications 2 à 8, caractérisé en ce qu'il renferme d'autres additifs cosmétiques tels que des produits de soin, des agents mouillants, des épaississants, des adoucissants, et des huiles parfumées.

**10.** Procédé de préparation du composé selon la revendication 1, caractérisé en ce qu'on fait réagir de la 4-fluoro-3-nitro-aniline sur du 3-amino-propanol, et l'on éthoxyle subséquemment la 4-(3'-hydropropyl)-amino-3-nitro-aniline formée, avec du 2-chloro-éthanol en présence d'une base.